# EUROPÄISCHE PATENTSCHRIFT

(11) **EP 1 139 863 B1**
(45) Veröffentlichungstag und Bekanntmachung des Hinweises auf die Patenterteilung: **11.09.2002**
(21) Anmeldenummer: 99966921.1
(22) Anmeldetag: 07.12.1999
(51) Int. Cl.: A61B 5/00

(54) **DETEKTIONSSONDE FÜR DIE TIEFENAUFLÖSENDE LICHT-SPEKTROSKOPIE UND SPEKTROMETRIE**
DETECTION PROBE FOR OPTICAL SPECTROSCOPY AND SPECTROMETRY
SONDE DE DETECTION POUR SPECTROSCOPIE ET SPECTROMETRIE OPTIQUE A RESOLUTION EN PROFONDEUR

(30) Priorität: 07.12.1998 DE 19856246
(43) Veröffentlichungstag der Anmeldung: 10.10.2001
(73) Patentinhaber: Lea Medizintechnik GmbH, 35394 Giessen (DE)
(72) Erfinder: OTT, Lutz, D-35463 Fernwald (DE); KRUG, Alfons, D-35390 Giessen (DE)
(74) Vertreter: Müller, Eckhard, Dr.
(86) Internationale Anmeldenummer: EP9909583
(87) Internationale Veröffentlichungsnummer: WO00033730

(56) Entgegenhaltungen:
- EP-A- 0 771 546
- DE-B- 2 517 129
- US-A- 4 321 930
- US-A- 5 259 382
- US-A- 5 299 570
- M COPE ET AL: "system for long-term measurement of cerebral blood and tissue oxygenation on newborn infants by near infra-red transillumination" MEDICAL AND BIOLOGICAL ENGINEERING AND COMPUTING,GB,PETER PEREGRINUS LTD. STEVENAGE, Bd. 26, Nr. 3, Mai 1988 (1988-05), Seiten 289-294-294, XP002105736 ISSN: 0140-0118
- WEIJIA CUI ET AL: "IN VIVO REFLECTANCE OF BLOOD AND TISSUE AS A FUNCTION OF LIGHT WAVELENGTH" IEEE TRANSACTIONS ON BIOMEDICAL ENGINEERING,US,IEEE INC. NEW YORK, Bd. 37, Nr. 6, 1. Juni 1990 (1990-06-01), Seiten 632-639, XP000136531 ISSN: 0018-9294

## Beschreibung

Die Erfindung bezieht sich auf Detektionssonden für die tiefenauflösende Licht-Spektroskopie und Spektrometrie.

Es gibt bereits verschiedene Möglichkeiten, um das im Gewebe gestreute Licht in Abhängigkeit vom Abstand zur Beleuchtungsquelle zu detektieren. Neben der Verwendung von Photodetektorarrays direkt auf der Gewebeoberfläche besteht die Möglichkeit, das Licht mit einer Anordnung von Lichtleitern zu den Detektoren zu übertragen.

Aus der US 4,321,930 ist eine Vorrichtung zum Erfassen von Metabolismen in Körpern bekannt, bei der eine Sonde auf die Haut aufgesetzt wird, die mehrere Umlenkeinrichtungen, nämlich gebogene Lichtleiter, enthält.

Ein entsprechendes Gerät ist als fotoelektrischer Pulsabnehmer mit Faseroptik auch aus der DE-A-25 17 129 bekannt.

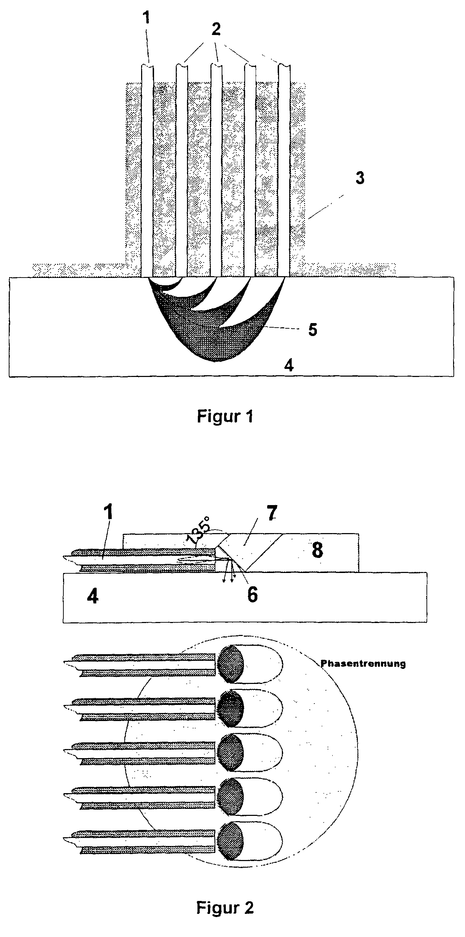

Bei einer bekannten Ausführung, wie sie in Fig. 1 dargestellt und in der EP 771 546 A2 beschrieben ist, können Lichtleitfasern in einer bestimmten Anordnung, die die Detektionstiefe und die Tiefenauflösung bestimmt, stumpf auf das Gewebe aufgesetzt werden. Dabei sind die Fasern (Beleuchtungsfaser 1, Detektionsfasern 2) in eine entsprechende Faserhalterung 3 (Material: z.B. PEEK, Edelstahl) eingeklebt oder mit einer Gießmasse (z.B. Epoxy-Gießmasse Epc-Tek 301-2) in vorher festgelegten Abständen zueinander fixiert. Aus Gründen der Biokompatibilität müssen entsprechende Werkstoffe verwendet werden. Gezeigt ist in Figur 1 weiterhin das Gewebe 4, in welches Licht aus der Faser 1 eingekoppelt wird, und die Wege 5 des gestreuten Lichtes, das von den einzelnen Detektionsfasern 2 aufgenommen wird. Wegen der verhältnismäßig ungünstigen Hebelverhältnisse muß eine solche Detektionssonde eine breite Basisplatte aufweisen, um sie sicher befestigen zu können. Durch die Verwendung von mehr als drei Fasern 1,2 wird das zuführende Faserkabel ziemlich steif, woraus große Biegeradii und damit ein großer Platzbedarf resultieren.

Durch die direkte Einkopplung des Streulichts in die Faser ist die Effektivität der bekannten Anordnung sehr hoch. Dies geht allerdings auf Kosten einer komfortablen Handhabung und Befestigung. Bei dieser Art der Lichtdetektion ist ein Faserdurchmesser beginnend von 50 µm bei einem Faserabstand von 0,3 mm ausreichend.

Demgegenüber ist es Aufgabe der Erfindung, die Baugröße der Detektionssonde zu verringern und die Handhabung zu verbessern.

Zur Lösung der Aufgabe ist eine Detektionssonde mit den Merkmalen des Anspruchs 1 vorgeschlagen. Ausführungen der Erfindung sind Gegenstand von Unteransprüchen.

Bevorzugt ist eine rechtwinklige Detektion des gestreuten Lichtes vorgesehen mit einer parallel zur Gewebeoberfläche geführten Faser und mit einer Umlenkung des Lichts zwischen 0° und 180°, bevorzugt um 90°, mit wenigstens einer Spiegelfläche.

Durch die Erfindung lassen sich die Detektionssonden besonders flach bauen, wodurch die Baugröße verringert und die Handhabung verbessert ist. Die Elektroden lassen sich ähnlich den Elektroden bei einem Elektrokardiogramm leicht befestigen und werden auch nicht als störend empfunden, da die "Zuleitungen" flach am Körper anliegen und nicht senkrecht davon wegstehen.

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Fig. 2, 3, 4, 5 und 6: jeweils Ausführungsformen erfindungsgemäßer Detektionssonden und
- Fig. 7: Sonden mit Befestigungsmöglichkeiten.

Bei dem ersten Ausführungsbeispiel gemäß Figur 2 wird die Lichtumlenkung durch Quarzfasern 7 erzielt. Dazu sind die Quarzfasern 1 und 2 für die Beleuchtung und die Detektion des rückgestreuten Lichtes wie bei der Anordnung gemäß Fig. 1 in eine Gießmasse 8 eingebettet, welche eine Faserhalterung 3 bildet.

Erfindungsgemäß sind die Fasern 1 und 2 aber nicht senkrecht zur Gewebeoberfläche geführt, sondern laufen im wesentlichen parallel zur Oberfläche des Gewebes 4. Um das Licht umzulenken, sind nun am Ende jeder Faser 1, 2 Quarzfasern 7 vorgesehen, die stirnseitig rechtwinklig geschliffen, poliert und anschließend verspiegelt sind. Diese Flächen bilden die Oberflächenspiegel 6, die das Licht aus der Faser 1 auf das Gewebe 4 umlenken und das aus der Tiefe des Gewebes rückgestreute Licht in die Fasern 2 umlenken. Diese Quarzfasern 7 werden unter einem Winkel von minimal 100° bis maximal 170°, bevorzugt von 135° gegen die Detektionsfasern 1, 2 mit diesen zusammen vergossen, so daß die Faserenden der Fasern 7 Spiegel bilden. Diese Anordnung kann in einem Gießvorgang gefertigt werden. Allerdings ist die Montage der Fasern 7 kritisch gegenüber Verschiebung, da die verspiegelten Fasern 7 nur wenig größer sind als die Detektionsfasern 1 und 2. Auf diese Weise lassen sich Sonden mit einer Höhe von etwa 3 mm fertigen. Der erforderliche Durchmesser hängt vom Faserabstand und damit von der gewünschten Tiefendetektion ab. Wegen des Abstands der Faserenden von der Gewebeoberfläche ist die räumliche Auflösung etwas reduziert, d.h. die detektierte Fläche ist entsprechend der numerischen Apertur der Faser und des Abstands zwischen Gewebeoberfläche und Faserende vergrößert. Bei der Reflexion an den verspiegelten Flächen geht etwas Licht verloren, was sich sowohl bei der Beleuchtung als auch bei der Detektion auswirkt.

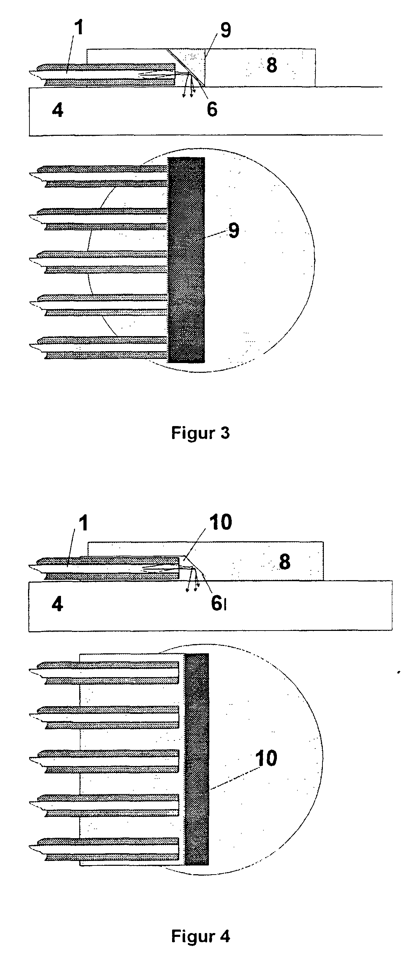

Bei dem zweiten Ausführungsbeispiel gemäß Figur 3 wird die Umlenkung des Lichts durch ein verspiegeltes Prisma 9 erzielt. Zur Vermeidung der gegen Verschiebung empfindlichen Justierung der Faserenden 7 gemäß dem ersten Ausführungsbeispiel ist ein verspiegeltes Prisma 9 vorgesehen. Das Prisma 9 wird in geringem Abstand von den Fasern 1, 2 mit diesen zusammen eingegossen. Der umlenkende Oberflächenspiegel 6 liegt an der den Fasern 1, 2 und dem Gewebe 4 zugewandten Seite des Glasprismas 9. Die Lichtumlenkung erfolgt an der Außenseite des Prismas 9. In Kenntnis dieser und der vorherigen Ausführung kann statt des Faserendes 7 oder des Prismas 9 auch ein anderer ebener Spiegel (nicht gezeigt) verwendet werden, der allerdings richtig justiert sein muß, was bei Verwendung des Prismas 9 einfacher ist.

Auch in dieser Ausführung ist zum Herstellen einer alle Bauteile haltenden Epoxy-Gießmasse 6 nur ein Gießvorgang erforderlich. Die Montageart mit Prisma ist justierunempfindlich, allerdings ist die Herstellung der Prismen 9 mit einer Kantenlänge von ca. 2 mm und einer Länge von 5-8 mm sehr aufwendig. Ansonsten weist diese Anordnung dieselben Eigenschaften auf wie diejenige nach dem ersten Ausführungsbeispiel gemäß Fig. 2.

Bei dem dritten Ausführungsbeispiel gemäß Figur 4 wird die Umlenkung des Lichts durch ein an die Faserenden der Fasern 1, 2 angegossenes Epoxy-Prisma 10 erzielt. Dabei werden die Fasern in gewünschtem Abstand mit überstehender Epoxy-Gießmasse quaderförmig vergossen. Das Ende dieses Epoxy-Blocks wird unter minimal 100° bis maximal 170°, bevorzugt unter 135°, gegenüber der Faserachse geschliffen, poliert und reflektiv beschichtet, wodurch der reflektierende Spiegel entsteht. Die Spiegelung erfolgt hier also innerhalb des Prismas 10. In einem zweiten Gießvorgang wird das Auflagescheibchen gegossen. Dies ist zum Schutz der spiegelnden Beschichtung erforderlich. Das Anschleifen des Prismas an den Epoxy-Quader ist sehr viel einfacher als das Schleifen eines Mini-Prismas aus Glas. Bei sorgfältiger Fertigung der Gießform kann sogar auf das Schleifen und Polieren der Spiegelfläche verzichtet werden.

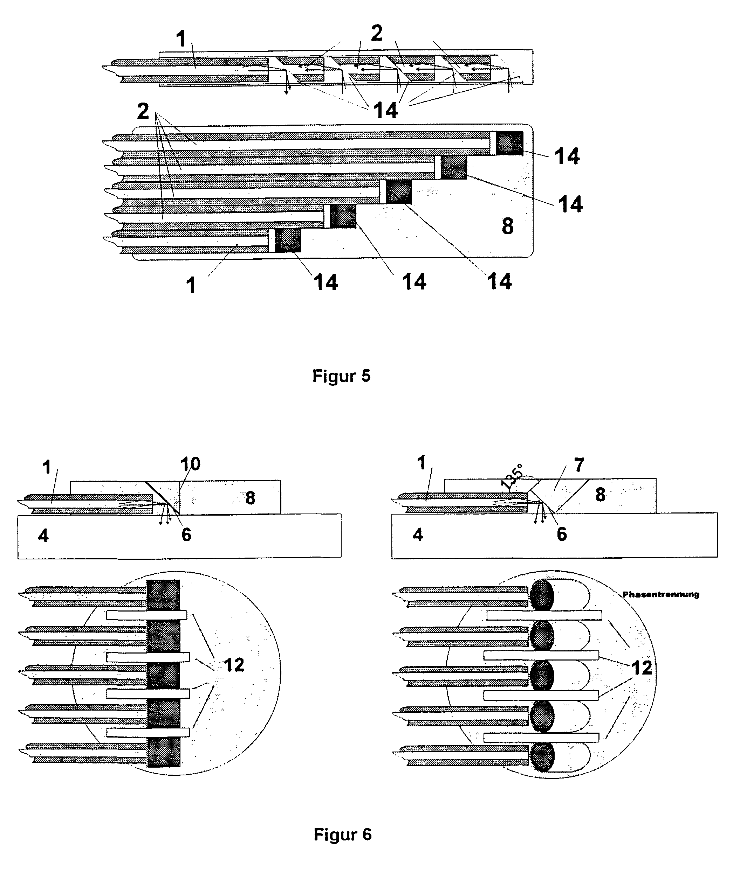

Bei dem vierten Ausführungsbeispiel gemäß Figur 5 wird die Umlenkung des Lichts durch mehrere angegossene Epoxy-Prismen 11 an versetzte Fasern 1 und 2 erzielt. Jedes der Prismen 11 enthält wiederum eine reflektierende Beschichtung.

Für den endoskopischen Einsatz muß die Sonde für die tiefenaufgelöste Detektion des Laser-Doppler-Signals miniaturisiert werden. Anzustreben ist ein Durchmesser < 3 mm, um den Arbeitskanal allgemein üblicher Endoskope nutzen zu können. Um die Tiefenauflösung zu gewährleisten, können die Abstände der Detektionsfasern 2 jedoch nicht beliebig verringert werden.

Bei den Faseranordnungen der Figuren 2 bis 4 ist der Durchmesser beziehungsweise die laterale Ausdehnung der Detektionssonde in erster Linie durch den Abstand zwischen den Fasern 2 bestimmt. Für relevante Detektionstiefen von einigen Millimetern kann ein Durchmesser von kleiner 2 mm nicht unterschritten werden. Damit sind diese Bauformen für den endoskopischen Einsatz nicht geeignet. Gemäß Fig. 5 kann der erforderliche Detektionsabstand durch axialen Versatz der Fasern 1, 2 erreicht werden. Dazu muß jeder Faser 1, 2 ein Prisma 11 angegossen werden. Dies kann bei einer geeigneten Gießform für alle Fasern 1, 2 in einem Arbeitsgang erfolgen. Die Prismen 11 müssen verspiegelt sein. Zum Schutz der reflektiven Schicht 14 muß das Faserbündel nochmals in einen dünnen Mantel eingegossen werden.

Die Figur 5 zeigt eine weitere Möglichkeit der Lichtumlenkung senkrecht zu den Fasern des Sensors, wobei mit dieser Technologie die verschiedensten Winkel realisierbar sind.

Die Technologie von Fig. 5 basiert auf der Möglichkeit, nahezu beliebige Lichtleiterstrukturen auf der Basis von PMMA-Materialien entwerfen zu können. Hiermit sind Umlenkwinkel gefertigt in Strukturtechnik möglich, die durch beschränkte Biegeradien der Fasern zuvor nicht realisierbar waren.

Die Ausführungsbeispiele nach den Fig. 2, 3, 4 werden durch ein Kammernsystem gemäß Fig.6 ergänzt, welches den ungewünschten Lichtübergang von einem zum nächsten Meßkanal schon unmittelbar im Sensorkopf verhindert. Entscheidend ist die Unterbrechung der Lichtverteilung von einem Kanal zu einem anderen Kanal zwischen den schrägstehenden, reflektierenden Flächen 6 (im linken Teilbild den Prismenflächen 10, im rechten Teilbild den Quarzfaserenden 7) durch einen guten Absorber 12 oder einen Reflektor. Wichtig ist zu vermeiden, daß Licht von einem Kanal zum anderen gelangt. Dies kann durch lichtundurchlässige Materialien erfolgen oder durch Spalte, die eine Lichtweiterleitung verhindern. Die Rückseite des Sensors besteht bevorzugt aus einem breitbandig hochabsorbierendem Material.

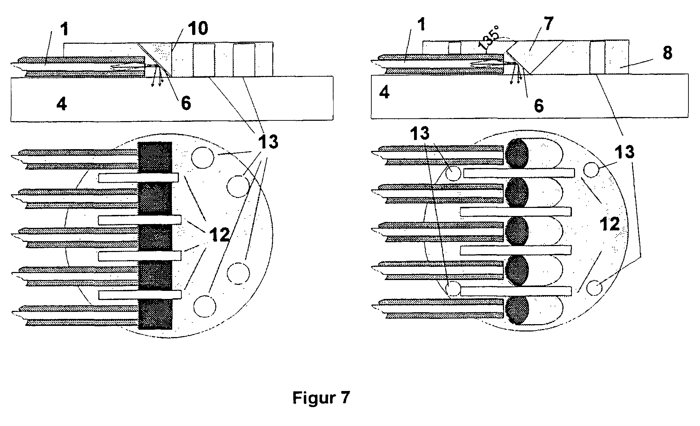

Entsprechend Fig. 7 können für Anwendungen in der Kardiologie und Inneren Medizin Löcher oder Ösen 13 am Sondenrand in der Gießmasse 8 angebracht sein, mit oder an denen der Sensor am Myocard oder anderen inneren Organen zur Befestigung aufgenäht wird. Die Anordnung der Löcher oder Ösen 13 ist frei wählbar.

Für den Einsatz in der klinischen Praxis ist es wünschenswert, auf den Einsatz des Fußschalters zum bewußten Einschalten der Laserleistung zu verzichten, um auch Langzeituntersuchungen durchführen zu können. Das hierfür entwickelte Sicherheitskonzept sieht vor, über zwei zusätzliche Kanäle unbedenklich anwendbares LED-Licht zu emittieren, das über eine Detektionsfaser wieder aufgefangen wird. Das ausgesandte LED-Licht kann nur detektiert werden, wenn der Sensorkopf am Gewebe aufliegt. Somit wird über diese Sicherheitsschaltung die Laserquelle nur dann eingeschaltet, wenn der Sensor auf dem Gewebe aufliegt und nicht, wenn er in den freien Raum emittiert und dabei unbeabsichtigt Schäden verursachen könnte. Bei den hier erforderlichen Sensoren muß eine entsprechende Anzahl von Kanäle integriert sein.

Der zusätzliche Vorteil dieser Ausführungsform liegt darin, daß die zu überwindenden Anforderungen für die CE-Zulassung sinken, da nun auf den Fußschalter verzichtet werden kann und der Sensorkopf nur noch optische und keine elektrischen Signale mehr beinhaltet. Hierzu fallen die Gerätschaften in eine niedrigere Schutzklasse bezüglich der zu erfüllenden Ableitströme.

## Patentansprüche

1. Detektionssonde für die Lichtspektroskopie und/oder Lichtspektrometrie in Gewebe mit Lichtleitfasern (1, 2), wobei mindestens eine Faser (1) Licht in das Gewebe (4) einleitet und andere Fasern (2) rückgestreutes Licht empfangen, **dadurch gekennzeichnet, daß** mehrere reflektierende Umlenkeinrichtungen, wie feste oder bewegliche Prismen (9, 10, 11), feste oder bewegliche Spiegel (6), feste oder bewegliche Linsen, angeschliffene Oberflächen, behandelte Lichtleitstrukturen, eigene Fasern (7) und/oder in Indexmaterialien geformte Lichtleitstrukturen, für das Licht vorgesehen sind, wobei der Lichtübergang von einer zur nächsten Umlenkeinrichtung durch die Anordnung einer oder mehrerer optischer Trennvorrichtungen (12) verhindert ist.

2. Detektionssonde nach Anspruch 1, **dadurch gekennzeichnet, daß** jede Faser (1, 2) eine Strahlumlenkung erfährt, wobei die Umlenkung im Bereich von 45° bis 135° erfolgt und wobei die zugeführten Fasern (1, 2) parallel zur Gewebeoberfläche geführt werden.

3. Detektionssonde nach einem der vorhergehenden Ansprüche, **dadurch gekennzeichnet, daß** für Laser-Doppler- und/oder gewebespektrometrische Messungen jede Strahlumlenkung eines Kanals unabhängig von den anderen ist, so daß kein optisches Übersprechen zwischen den Meßkanälen durch getrennte Strahlführung auftritt.

4. Detektionssonde nach einem der vorhergehenden Ansprüche, **dadurch gekennzeichnet, daß** zwei Fasern des Sensors mitbenutzt werden für die Bestimmung des Sensorabstandes zum Gewebe, wobei als Sendelichtquelle amplitudenmoduliertes und/oder phasenmoduliertes Licht eingesetzt wird.

## Claims

1. Detection probe for light spectroscopy and/or light spectrometry in tissue by optical conductors (1, 2), wherein at least one fibre (1) conducts light into the tissue (4) and other fibres (2) receive backscattered light, **characterised in that** several reflective deflecting devices, such as fixed or movable prisms (9, 10, 11), fixed or movable mirrors (6), fixed or movable lenses, ground surfaces, treated optical conductor structures, own fibres (7) and/or optical structures formed in index materials, are provided for the light, wherein light transfer from deflecting device to the next is prevented by arrangement of one or more optical separating devices (12).

2. Detection probe according to claim 1, **characterised in that** each fibre (1, 2) experiences a beam deflection, wherein the deflection takes place in the range of 45 to 135° and wherein the supplied fibres (1, 2) are guided parallel to the tissue surface.

3. Detection probe according to one of the preceding claims, **characterised in that** for laser/Doppler measurements and/or tissue-spectrometric measurements each beam deflection of a channel is independent of the others, so that no optical cross-talk between the measuring channels arises due to separate beam guidance.

4. Detection probe according to one of the preceding claims, **characterised in that** two fibres of the sensor are jointly used for determination of the sensor spacing from the tissue, wherein amplitude-modulated and/or phase-modulated light is used as transmission light source.

## Revendications

1. Sonde de détection pour spectroscopie optique et/ou spectrométrie optique dans un tissu comportant des fibres optiques (1, 2), au moins une fibre optique (1) introduisant la lumière dans le tissu (4), et d'autres fibres optiques (2) recevant la lumière rétrodiffusée, **caractérisée en ce que** plusieurs dispositifs de déviations réfléchissants, comme par exemple des prismes fixes ou mobiles (10, 11), des miroirs fixes ou mobiles (6), des lentilles fixes ou mobile, des surfaces polies, des structures traitées de guidage de la lumière, des fibres proprement dites (7) et/ou des structures de guidage de la lumière réalisées avec des matériaux à gradient d'indice, sont prévus pour la lumière, et dans lesquels le transfert de la lumière d'un dispositif de déviation au dispositif de déviation suivant est empêché grâce à l'agencement d'un ou de plusieurs dispositifs de séparation optiques (12).

2. Sonde de détection selon la revendication 1, **caractérisée en ce que** chaque fibre (1, 2) est le siège d'une déviation du rayonnement, la déviation s'effectuant dans la gamme de 45° à 135° et les fibres d'arrivée (1, 2) étant guidées parallèlement à la surface du tissu.

3. Sonde de détection selon l'une des revendications précédentes, **caractérisée en ce que** pour des mesures Doppler à laser et/ou des mesures spectrométriques du tissu, chaque déviation du rayonnement d'un canal est indépendant de celle de l'autre canal, de sorte qu'il n'apparaît aucune diaphonie optique entre les canaux de mesure en raison d'un guidage séparé du rayonnement.

4. Sonde de détection selon l'une des revendications précédentes, **caractérisée en ce que** deux fibres du capteur sont utilisées conjointement pour la détermination de la distance entre le capteur et le tissu, une lumière modulée en amplitude et/ou modulée en phase étant utilisée en tant que source de lumière d'émission.
